# EUROPEAN PATENT APPLICATION

(11) **EP 1 518 572 A1**
(43) Date of publication of application: **30.03.2005**
(21) Application number: 04255762.9
(22) Date of filing: 22.09.2004
(51) Int. Cl.: A61M 1/10, F04B 43/12

(54) **Tube pump**

(30) Priority: 29.09.2003 JP 2003337150
(71) Applicant: Japan Servo Co. Ltd., Chiyoda-Ku, Tokyo-to (JP)
(72) Inventor: Sibasaki, Masanori Japan Servo Co. Ltd., Kiryu-shi Gunma-ken (JP)
(74) Representative: Naylor, Matthew John

(57) **Abstract**

Disclosed is a tube pump (1) including a base (20) that holds a drive shaft (21); a rotor portion (30) that rotates with the driving shaft, the rotor portion having a pair of rollers (34a,34b); a casing (40) having a U-shaped internal surface (41) along which an elastic tube (c) is arranged, the casing (40) being mounted on the base (20) so that the casing (40) can slide with respect to the base (20) in a direction parallel to the linear portions (41b,41c); a cover (50) that is rotatably mounted on the casing (40); a moving mechanism including a rack (25) that is formed on the base (20) and a pinion (45) that is fixed to the rotation shaft (44) of the cover (50). The moving mechanism moves the casing (40) to the operating position where the roller (34a,34b) deforms the elastic tube (c) when the cover (50) closes, and the moving mechanis moves the casing (40) to the exchange position where the roller (34a,34b) comes apart from the elastic tube (c) when the cover (50) opens.

## Description

The present invention relates to a tube pump that deforms an elastic tube by rotating rollers to send out liquid in the elastic tube. Especially the present invention relates to a tube pump mounted on artificial dialysis equipment etc. that enables easy attachment and removal of the elastic tube.

This kind of tube pump is disclosed in Japanese unexamined patent publication No. Hei 6-218042, for example. The tube pump disclosed in the publication is provided with a casing body having a semicircular internal surface, a drive shaft located at the center of the semi-circle and a rotor portion that rotates with the drive shaft. The rotor portion has a pair of rollers whose rotation axes are decentered from the drive shaft. For operation, arrange an elastic tube along the internal surface of the casing body and rotate the rotor portion to deform the elastic tube with compression between the internal surface and the rollers to send liquid in the elastic tube.

Since the tube pump sends liquid by deforming the elastic tube, the tube tends to deteriorate, which requires frequent exchange of the tube. Further, when this kind of tube pump is used in artificial dialysis equipment, the drawing direction of the elastic tube may be changed to match layout of the artificial dialysis equipment with respect to a bed of a patient or to avoid frequently inserting shunt needles into the same arm of a patient. However, since the elastic tube is pinched between one roller and the internal surface of the tube pump, it takes much expense in time and effort to exchange the tube even if the rotor portion stops.

Therefore, the tube pump of the above publication divides the casing body into a movable casing that forms a part of the semicircular internal surface and a fixed casing that forms the other portion. This enables to exchange the tube under the condition where the roller comes apart from the elastic tube by separating the movable casing from the fixed casing. With this construction, the elastic tube can be easily attached and removed.

However, since the tube pump disclosed in the above publication divides the semicircular internal surface to which large pressure is applied by the rotation of the rotor portion in operation into two portions, it is difficult to keep structural strength. Therefore, the movable and fixed casings are formed from metal, which increases the cost of the tube pump in comparison with that with plastic casings. Although the publication describes that the casings may be made fromplastic, if the casings having the same structure are made from plastic, there is a high probability that the casings break due to lack of strength in reality.

Further, the tube pump of the above publication slides the movable casing in response to the opening and closing operations of the casing cover. However, since the casing to which large pressure is applied is divided as described above, a solenoid to lock the movable casing at the position connecting to the fixed casing and a sensor that detects the opening and closing of the cover to control the solenoid are required, which complicates the construction of the tube pump.

Still further, the tube pump of the publication has a pair of tube clamp arms that hold the tube at the entrance and exit positions of the pump. However, since the clamp arms must be operated manually, the operation of the clamp arms for exchanging the tube is complicated.

A first object of the present invention is to provide a tube pump that can increase structural strength of a casing so that it can be made from plastic with easy attachment and removal of an elastic tube.

A second object of the present invention is to provide a tube pump that does not require a mechanism for locking a casing at an operating position on the precondition that a roller changes its position in response to opening/closing of a cover between the operating position where the roller deforms the elastic tube and an exchange position where the roller comes apart from the elastic tube.

A third object of the present invention is to provide a tube pump that can automatically operate clamp arms arranged at entrance and exit positions of the pump.

In order to address the first and second objects, a tube pump of a first aspect of the present invention includes:
a base that holds a drive shaft that connects with and is driven by a motor;
a rotor portion that rotates with the driving shaft, the rotor portion having a pair of rollers each of which has a rotating shaft decentered from the drive shaft;
a casing having a U-shaped internal surface that includes a semicircular portion and parallel linear portions connected to both ends of the semicircular portion along which an elastic tube is arranged, the casing being mounted on the base so that the casing can slide with respect to the base in a direction parallel to the linear portions;
a cover that is rotatably mounted on the casing at the outside of the semicircular portion, the cover covering the portion surrounded by the internal surface at its closing position;
a moving mechanism including a rack that is formed on the base and a pinion that is fixed to the rotation shaft of the cover and engages with the rack, the moving mechanism moving the casing with respect to the base to an operating position where the roller deforms the elastic tube arranged along the internal surface when the cover closes, and the moving mechanism moving the casingwith respect to the base to an exchange position where the roller comes apart from the elastic tube when the cover opens.

With this construction, since the casing to which large pressure is applied is not separated, the structural strength becomes larger, which keeps the casing intact even if it is made from plastic. Further, when an elastic tube is located along the internal surface of the casing and the cover is closed, the rotation of the rotor portion deforms the elastic tube between the roller and the internal surface to send out liquid in the elastic tube. When the cover is opened, the elastic tube can be easily exchanged.

A step portion (a level difference portion) may be formed on the internal surface of the casing so that the diameter of the semicircular portion at the side of the cover becomes larger than the other side. Further, a convex rim, which can insert inside the internal surface, is formed on the cover at the position opposite to the step portion. The step portion and the convex rim function as a means for guiding the elastic tube that is arranged along the internal surface.

In order to address the above-described third object, a tube pump of a second aspect of the present invention has the following characteristic features. That is, the base is provided with a pair of clamp arms that hold entrance and exit portions of the elastic tube to the pump, respectively, the base end of each clamp arm is rotatably attached to a pivot that is vertical to the axis of the drive shaft and is parallel to the linear portions, each clamp arm has a tip end that is formed to hold the elastic tube and has a small roller at a middle portion thereof. The base has a biasing mechanism that applies bias pressure to the clamp arms so that the small rollers move closer and the clamp arms release the elastic tube. A connecting projection, which intrudes between the small rollers when the cover closes, is formed on the cover.

With this construction, when the cover opens, since the tip ends of the clamp arms release the elastic tube due to the bias pressure applied by the biasing mechanism, the elastic tube can be easily exchanged. When the cover closes, the connecting projection of the cover intrudes between the small rollers and separates them from each other, which rotates the clamp arms against the bias pressure applied by the biasing mechanism. Therefore, the tip ends of the clamp arms are pressed to the elastic tube and the clamp arms hold the elastic tube.

As described above, since the elastic tube is automatically held and released in response to the opening and closing of the cover without manual operation of the clamp arms, the elastic tube can be easily exchanged.

In addition, a permanent magnet may be mounted on the rotor portion at the position opposite to the casing and a magnetic sensor may be installed on either of the casing and the base to determine the rotation position of the rotor portion by detecting the magnetic flux of the permanent magnet. The output of the magnetic sensor is used to control a motor that drives the rotor portion. For instance, when the rotation of the rotor portion is stopped, the motor is controlled to stop one roller at the midpoint of the semicircular portion of the casing so that the movement of the casing by the moving mechanism can come apart the roller from the elastic tube. For easy exchange of the elastic tube, the roller must come apart from the elastic tube at the exchange position. If the rotor portion stops at the position where the roller faces the linear portion of the internal surface, since the roller does not come apart from the elastic tube when the cover opens, it becomes difficult to exchange the elastic tube. Therefore, it is preferable to predetermine the stop position and to control the motor for driving the tube pump according to the predetermined stop position.

By way of example ,the invention will now be described in greater detail with reference to the accompanying drawings of which:
Fig. 1 is a perspective plan view of a tube pump of an embodiment according to the present invention when an elastic tube is attached to the tube pump and a cover closes;
Fig. 2 is a sectional side view of Fig. 1 along the II-II line;
Fig. 3 is a sectional front view of Fig. 1 along the III-III line;
Fig. 4 is a plan view of the tube pump of the embodiment when the cover opens before the elastic tube is removed;
Fig. 5 is a plan view of the tube pump of the embodiment when the cover opens after the elastic tube and the rotor portion are removed;
Fig. 6 is a sectional side view of Fig. 4 along the VI-VI line;
Fig. 7 is a sectional view of Fig. 1 along the VII-VII line when a tube clamp mechanism holds the elastic tube;
Fig. 8 is a sectional view of Fig. 4 along the VIII-VIII line when the tube clamp mechanism releases the elastic tube; and
Fig. 9 is a system chart showing the entire construction of artificial dialysis equipment that employs the tube pump of the embodiment as a blood pump.

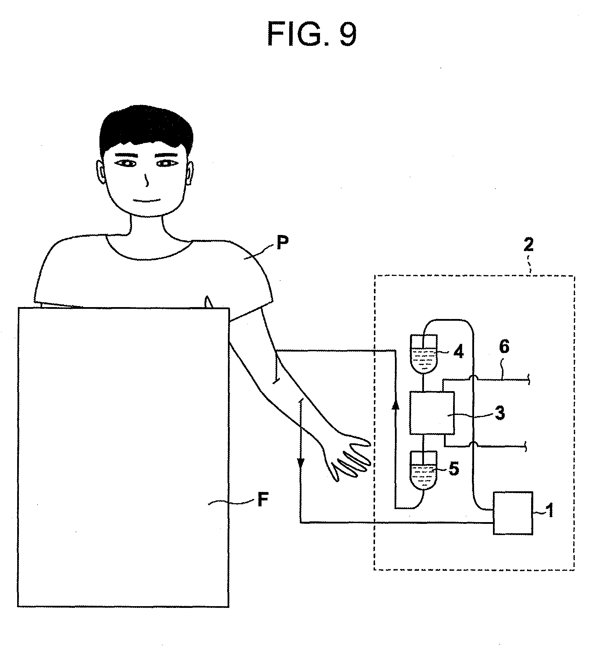

Hereinafter, a tube pump of an embodiment according to the present invention will be described with reference to the drawings. Initially, an example of the use of the tube pump of the embodiment will be described according to Fig. 9.

Fig. 9 shows the entire construction of artificial dialysis equipment 2 that employs the tube pump 1 of the embodiment as a blood pump. The artificial dialysis equipment 2 is provided with the tube pump 1, a dialyzer 3, first and second chambers 4 and 5. The equipment 2 is incorporated in an extracorporeal circulation path of a patient P. In addition, the reference F in Fig. 9 indicates a cover sheet put on the body of the patient P.

Blood collected from the arm of the patient P is drawn by the tube pump 1 through the tube and is pressurized to send to the first chamber 4. The blood that is temporally accumulated in the first chamber 4 is sent to the dialyzer 3. The dialyzer 3 dialyzes and filters the blood with using a dialyzate solution supplying line 6 to remove deleterious material and excess water. The blood after dialysis is temporally accumulated in the second chamber 5 and then is sent back to the arm of the patient P.

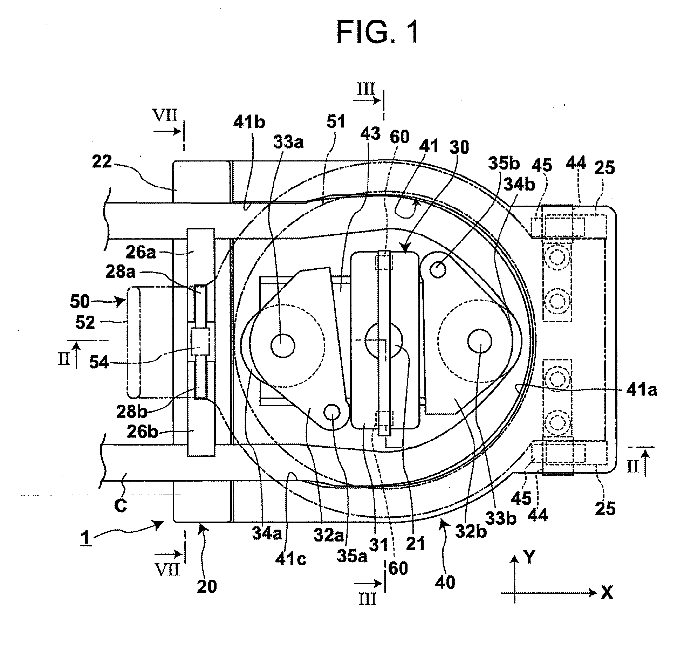

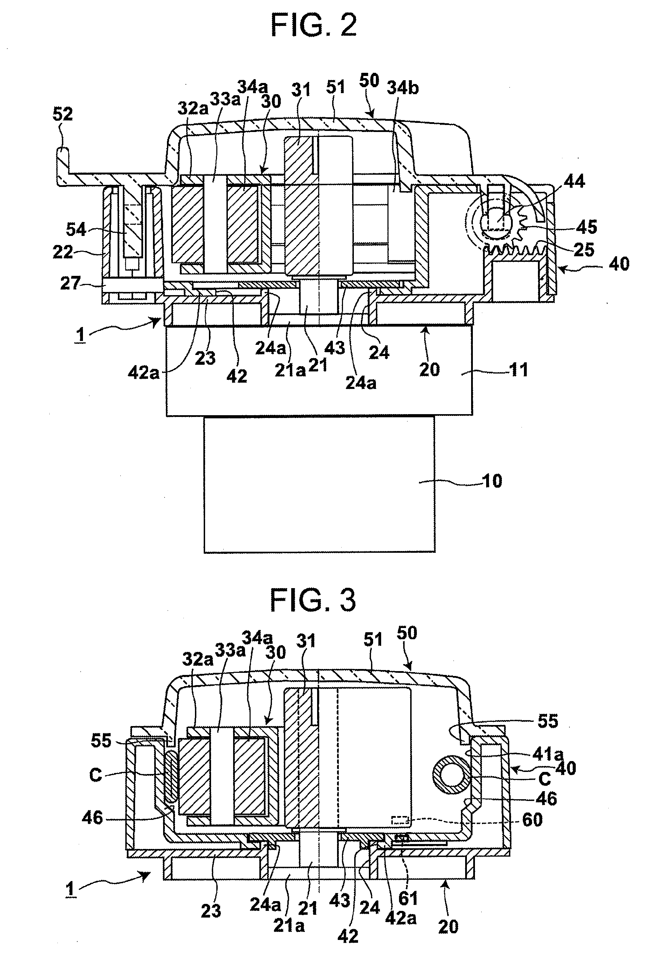

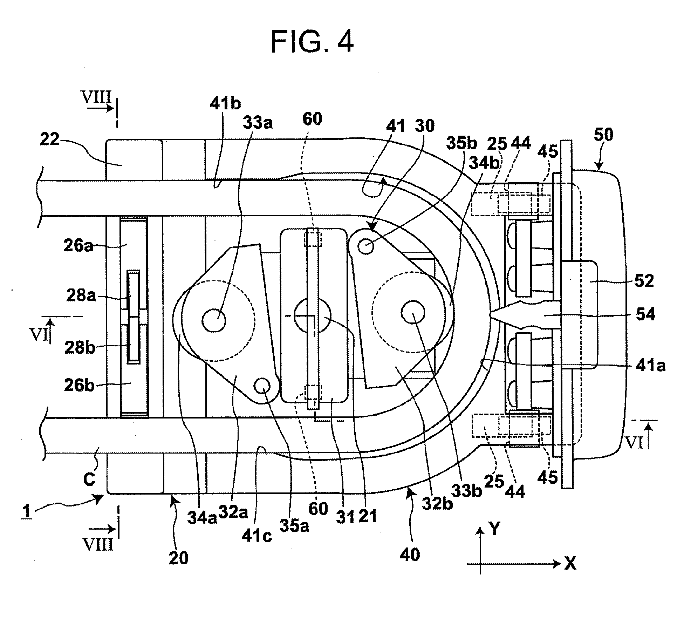

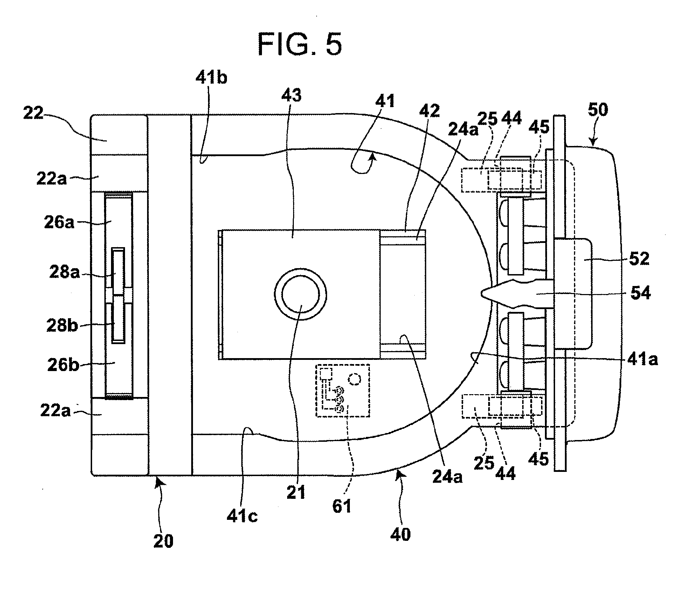

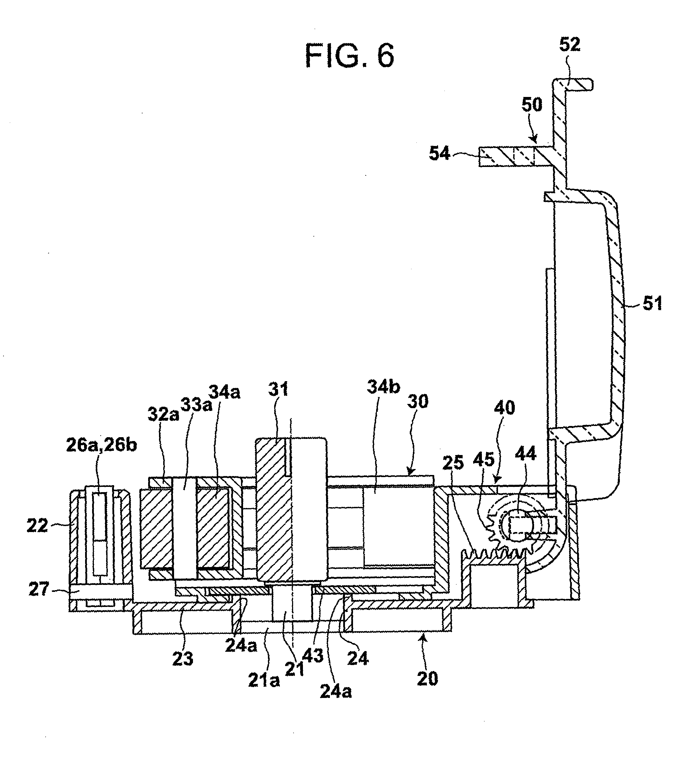

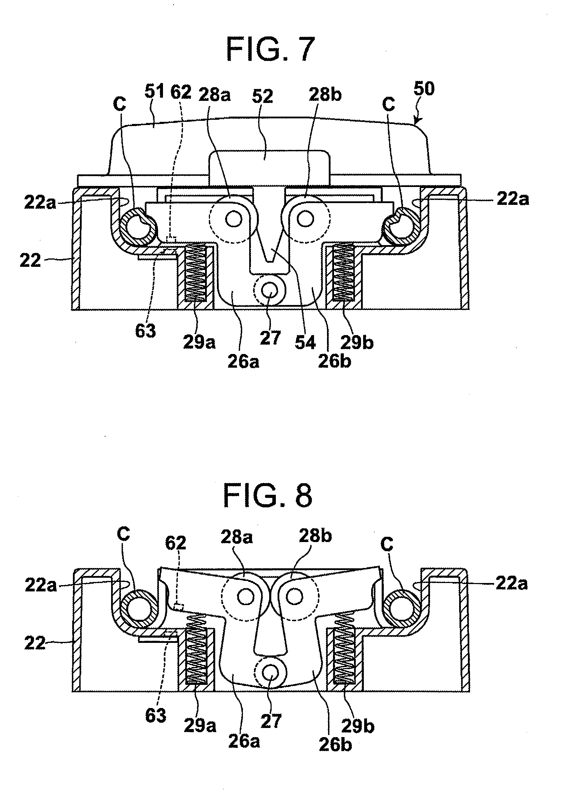

The construction of the above-described tube pump 1 will be described with reference to Fig. 1 through Fig. 8. Fig. 1 through Fig. 3 show the tube pump 1 of the embodiment when the elastic tube C is placed and the cover closes, Fig. 1 is a perspective plan view (the cover is illustrated by the two-dot chain line), Fig. 2 is a sectional side view of Fig. 1 along the II-II line and Fig. 3 is a sectional front view of Fig. 1 along the III-III line (a position of a rotor portion is different from that in Fig. 1). Fig. 4 through Fig. 6 show the tube pump 1 of the embodiment when the cover opens, Fig. 4 is a plan view before the elastic tube is removed, Fig. 5 is a plan view after the elastic tube and a rotor portion shown in Fig. 4 are removed and Fig. 6 is a sectional side view of Fig. 4 along the VI-VI line. Fig. 7 and Fig. 8 show a tube clamp mechanism that holds the elastic tube C at the entrance and exit positions of the tube pump 1, Fig. 7 is a sectional view of Fig. 1 along the VII-VII line and Fig. 8 is a sectional view of Fig. 4 along the VIII-VIII line.

The tube pump 1 of the embodiment is provided with, as shown in Fig. 1 and Fig. 2, a base 20 having a drive shaft 21 that is connected to a motor 10 through a reduction gear 11, a rotor portion 30 attached to the drive shaft 21, a casing 40 that can slide in an arrowedX-direction in Fig. 1 with respect to the base 20 and a cover 50 that covers over the casing 40. When the drive shaft 21 is rotated by the motor 10, the rotor portion 30 rotates together with the drive shaft 21.

The base 20 includes a fixing portion 22 for fixing the elastic tube C at the entrance and exit positions and a holding portion 23 for holding the casing 40 so that the casing 40 can slide in the X-direction. At the center of the holding portion 23, a base opening 24 is formed for allowing the drive shaft 21 to pass through. A bearing 21a for supporting the drive shaft 21 is installed in the base opening 24.

The rotor portion 30 is a single unit that is detachable/attachable to the drive shaft 21, and it consists of a rotor body 31 having a shape of a rectangular parallelepiped to which the drive shaft 21 is inserted, a pair of roller holders 32a, 32b attached to both sides of the rotor body 31, and a pair of rollers 34a, 34b that are supportedby the roller holders 32a, 32b via rotating shafts 33a, 33b decentered from the drive shaft 21. The roller holders 32a and 32b are connected to the rotor body 31 by axial pins 35a and 35b mounted on diagonal positions of the rotor body 31 so that the roller holders 32a and 32b can pivot about the axial pins 35a and 35b, respectively. Further, the free ends of the respective roller holders 32a and 32b are pushed by compression springs (not shown) in the direction that distances the free ends from the rotor body 31.

There is a space at the center of the casing 40 for arranging the rotor portion 30 and the elastic tube C, and a sidewall surrounds the space from three directions. A U-shaped internal surface 41 of the sidewall consists of a semicircular portion 41a and a pair of linear portions 41b, 41c that are connected to both ends of the semicircular portion 41a. The elastic tube C is located along the internal surface 41.

Further, the casing 40 is mounted on the base 20 so that the casing 40 can slide in the X-direction in Fig. 1, which is the direction of the linear portions 41b, 41c, with respect to the base 20. That is, as shown in Fig. 2, Fig. 3 and Fig. 5, a projecting edge 24a is formed along the upper edge of a base opening 24 formed on the base 20 and a casing opening 42 is formed on the bottom of the casing 40 to connect with the base opening 24. The casing opening 42 is larger than the base opening 24 in the X-direction as shown in Fig. 2 and contacts the outside of the projecting edge 24a of the base opening 24 in a Y-direction as shown in Fig. 3. A flange 42a is formed on the edge of the casing opening 42 and the flange 42a, which is stepped down from the casing opening 42, is sandwichedbetween the base 20 and a fixing plate 43 that is fitted in the base opening 42. With this construction, the casing 40 can slide in the X-direction with respect to the base 20 without changing its position in the Y-direction and is held by the fixing plate 43 not to be removed.

In addition, the base 20, the casing 40 and the rotor body 31 of the rotor portion 30 are made from rigid plastic. The rotor holders 32a and 32b aremade frommetal or rigidplastic.

As shown in Fig. 1 and Fig. 2, the cover 50, which is made from transparent plastic, is provided with a dome portion 51 that covers the inside of the internal surface 41 of the casing body 40 and is fixed by screws to the rotation axis 44 that is rotatably mounted on the one end of the casing 40. On the other end of the cover 50, a knob 52 is formed to be handled by an operator for closing and opening operations. The knob 52 has a connecting projection 54 that is formed at the inside of the knob 52 towards the casing 40 to connect with the fixing portion 22 of the base 20. The function of the connection projection 54 will be described below.

Next, the moving mechanism that moves the casing 40 with respect to the base 20 will be described. As shown in Figs. 2 and 6, a rack 25 is formed on one side of the base 20 and a pinion 45 that engages the rack 25 is fixed to a rotation shaft 44 that rotates with the opening/closing of the cover 50. As shown in Figs. 1 and 4, the pinions 45 are fixed to both sides of the rotation shaft 44 and the racks 25 are formed at the corresponding positions.

The opening/closing operations of the cover 50 rotates the pinions 45, which changes the relative position between the pinions 45 and the racks 25 in the X-direction, sliding the casing 40 with respect to the base 20. When the cover 50 closes as shown in Fig. 1, the casing 40 moves the roller 34b with respect to the base 20 to an operating position where the roller 34b deforms the elastic tube C arranged along the internal surface 41. When the cover 50 opens as shown in Fig. 4, the casing 40 moves roller 34b with respect to the base 20 to an exchange position where the roller 34b comes apart from the elastic tube C to release the deformation.

As shown in Fig. 3, a step portion (a level difference portion) 46 is formed on the semicircular portion 41a of the internal surface 41 of the casing 40 so that the diameter at the side of the cover 50 (the upper side in Fig. 3) becomes larger than the other side (the lower side) . Further, a convex rim 55, which can insert inside the larger diameter portion of the internal surface 41, is formed on the cover 50 at the position opposite to the step portion 46. The step portion 46 and the convex rim 55 function as a guiding means to prevent up-and-down slippage of the elastic tube C that is arranged along the internal surface 41.

Next, the structure of the fixing portion 22 of the base 20 will be described with reference to Fig. 7 and Fig. 8. A pair of U-shaped grooves 22a are formed on the fixing portion 22 to guide entrance and exit portions of the elastic tube C and a pair of clamp arms 26a, 26b to hold the elastic tube C arranged in the U-shaped grooves 22a are provided. The clamp arms 2 6a, 2 6b have crank-shapes that are symmetric to each other. The base ends of the clamp arms 26a, 26b are rotatably attached to a pivot 27 that is vertical to the axis of the drive shaft 21 and is parallel to the linear portions 41b, 41c of the internal surface 41. Further, the respective clamp arms 26a, 26b have the tip ends providing circular cutout portions to hold the elastic tube C and have small rollers 28a, 28b at middle portions. Still further, the base 20 has a pair of coil springs 29a, 29b as a biasing mechanism that applies bias pressure to the clamp arms 26a, 26b so that the small rollers 28a, 28b move closer and the clamp arms 26a, 26b release the elastic tube C.

When the cover 50 opens, the tip ends of the clamp arms 26a, 26b release the elastic tube C due to the bias pressure applied by the coil springs 29a, 29b as shown in Fig. 8. When the cover 50 closes, the connecting projection 54 of the cover 50 intrudes between the small rollers 28a, 28b and separates them from each other as shown in Fig. 7, which rotates the clamp arms 26a, 26b so that the tip ends hold the elastic tube C to fix the elastic tube C to the fixing portion 22 against the bias pressure applied by the coil springs 29a, 29b.

In addition, a pair of permanent magnets 60 are installed at the lower edge of the rotor body 31 at the positions indicated by the dotted lines in Fig. 1 and Fig. 3. A rotation position sensor 61, which has a magnetic sensor such as a hall element, is mounted on the back surface of the casing 40 in the space between the casing 40 and the base 20. The rotation position sensor 61 is located so that it can detect the magnetic flux of the permanent magnets 60.

On the other hand, a permanent magnet 62 is installed on the lower end of the clamp arm 26a and an arm position sensor 63 having a magnetic sensor is mounted on the fixing portion 22 to detect the magnetic flux of the permanent magnet 62.

The outputs of the sensors 61 and 63 are checked by a controller (not shown). The output of the rotation position sensor 61 is used to detect the rotation position of the rotor portion 30 during operation of the tube pump 1. The output of the arm position sensor 63 is used to determine whether the cover 50 opens or closes.

Next, the function of the tube pump 1 that is constructed as above will be described. During operation of the tube pump 1, an operator arranges the elastic tube C along the internal surface 41 of the casing 40, closes the cover 50 and turns on a operation switch (not shown) . When the cover 50 is closed, as shown in Fig. 1 and Fig. 2, the casing 40 relatively moves leftward in the drawings with respect to the base 20 to the operating position where the roller 34b deforms the elastic tube C. Further, the connecting projection 54 of the cover 50 intrudes between the small rollers 28a, 28b, which rotate the clamp arms 26a, 26b to hold the elastic tube C to the fixing portion 22 at the entrance and exit points as shown in Fig. 7 . The controller checks the output from the armposition sensor 63. When the controller determines that the cover 50 closes and the clamp arm26aholds the elastic tube C, it applies electric current to the motor 10 to rotate. The rotation of the motor 10 is transferred via the reduction gear 11 to the drive shaft 21, which rotates the rotor portion 30 attached to the drive shaft 21. Assuming that the rotor portion 30 rotates in the counterclockwise direction in Fig. 1, the roller 34a contacts the elastic tube C when the roller 34a comes the position opposite to the linear portion 41c of the internal surface 41. The roller 34a revolves both on the rotating shaft 33a and around the drive shaft 21 with deforming the elastic tube C by the pressure caused by the compression spring (not shown) arranged between the roller holder 32a and the rotor body 31. The elastic tube C is squeezed as the roller 34a advances to the semicircular portion 41a and the linear portion 41b, the liquid in the tube flows in the counterclockwise direction and is pushed out to the exit side. The other roller 34b contacts the elastic tube C just before the roller 34a comes apart from the elastic tube C. The roller 34b sends out the liquid in the tube in the same manner as the roller 34a. With this construction, since the rollers 34a and 34b alternately squeeze the elastic tube C, the tube pump 1 can continuously send out the liquid.

When an operator stops the tube pump 1, the operator turns off the operation switch. The controller detects the output signal from the rotation position sensor 61 and continues to apply electric current until a predetermined time elapsed after detecting the output signal from the rotation position sensor 61, and then cuts the electric current. The predetermined time is an interval from the detection of the output signal of the rotation position sensor 61 to the arrival of the rotor portion 30 to the position where the rollers 34a and 34b align in the X-direction as shown in Fig. 1.

As described above, since the rotor portion 30 is controlled so that one roller stops at the midpoint of the semicircular portion 41a of the casing 40, the rollers 34a and 34b come apart from the elastic tube by the movement of the moving mechanism, which allows easy exchange of the elastic tube.

When the elastic tube C is exchanged, the operator stops the rotation of the rotor portion 30 and open the cover 50. Opening the cover 50 moves the casing 40 rightward in Fig. 4 and Fig. 6 with respect to the base 20, which locates the roller 34b apart from the elastic tube C. Further, since the connecting projection 54 of the cover 50 gets out of the position between the small rollers 28a and 28b as shown in Fig. 8, the clamp arms 26a and 26b rotate by the tension of the compression springs 29a and 29b, which moves the tip ends off the elastic tube C. Under this condition, the elastic tube C can be easily removed and exchanged.

With the above-described structure, since the casing 40 to which large pressure is applied is not separated, the structural strength becomes larger, which keeps the casing intact even if it is made from plastic. Further, since the casing 40 moves and the clamp arms 26a, 26b rotate in response to the opening/closing operations of the cover 50, the elastic tube C can be easily exchanged.

## Claims

1. A tube pump comprising:
a base that holds a drive shaft that connects with and is driven by a motor;
a rotor portion that rotates with said driving shaft, said rotor portion having a pair of rollers each of which has a rotating shaft decentered from said drive shaft;
a casing having a U-shaped internal surface that includes a semicircular portion and parallel linear portions connected to both ends of said semicircular portion along which an elastic tube is arranged, said casing being mounted on said base so that said casing can slide with respect to said base in a direction parallel to said linear portions;
a cover that is rotatably mounted on said casing at the outside of said semicircular portion, said cover covering the portion surrounded by said internal surface at its closing position;
a moving mechanism including a rack that is formed on said base and a pinion that is fixed to a rotation shaft of said cover and engages with said rack, said moving mechanism moving said casing with respect to said base to an operating position where said roller deforms said elastic tube arranged along said internal surface when said cover closes, and said moving mechanism moving said casing with respect to said base to an exchange position where said roller comes apart from said elastic tube when said cover opens.

2. The tube pump according to claim 1, wherein said internal surface of said casing has a step portion so that the diameter of the semicircular portion at the side of the cover becomes larger than the other side, and said cover has a convex rim, which can insert inside said internal surface, is formed on said cover at the position opposite to said step portion, whereby said step portion and said convex rim function as a means for guiding said elastic tube that is arranged along said internal surface.

3. The tube pump according to claim 1 or 2, further comprising:
a pair of clamp arms that is mounted on said base to hold entrance and exit portions of said elastic tube to the pump, respectively, the base end of each of said clamp arm being rotatably attached to a pivot that is vertical to the axis of said drive shaft and is parallel to said linear portions, each of said clamp arm having a tip end that is formed to hold said elastic tube and having a small roller at amiddleportion thereof;
a biasing mechanism that applies bias pressure to said clamp arms so that said small rollers move closer and said clamp arms release said elastic tube; and
a connecting projection that is formed on said cover to intrude between said small rollers when said cover closes,
wherein said connecting projection of the cover intrudes between said small rollers and separates them from each other, which rotates the clamp arms against the bias pressure applied by the biasing mechanism when the cover closes.

4. The tube pump according to one of claims 1 through 3, further comprising:
a permanent magnet mounted on said rotor portion at the position opposite to the casing; and
a magnetic sensor installed on either of said casing and said base for detecting magnetic flux of said permanent magnet to determine the rotation position of said rotor portion.
